# EUROPEAN PATENT APPLICATION

(11) **EP 4 488 356 A1**
(43) Date of publication of application: **08.01.2025**
(21) Application number: 23784776.9
(22) Date of filing: 05.04.2023
(51) Int. Cl.: C12M 1/00, C12M 1/26, C12Q 1/02

(54) **CELL SEEDING DEVICE, INFORMATION PROCESSING DEVICE, AND INFORMATION PROCESSING METHOD**

(30) Priority: 06.04.2022 JP 2022063641
(71) Applicant: FUJIFILM Corporation, Tokyo 106-8620 (JP)
(72) Inventor: SHIRAISHI, Yasushi, Ashigarakami-gun, Kanagawa 258-8577 (JP)
(74) Representative: Hoffmann Eitle
(86) International application number: PCT/JP2023/014107
(87) International publication number: WO 2023/195492

(57) **Abstract**

A cell seeding apparatus of the present disclosure includes a dropping device that drops a liquid droplet of a cell suspension into a cell culture vessel having a measurement electrode and a reference electrode disposed on a bottom surface, an imaging device that is provided in the dropping device and that images the bottom surface to generate an image, and an information processing device that detects positions of the measurement electrode and the reference electrode based on the image and that decides, based on detection information, a dropping position of the liquid droplet at which cells are to be placed on the measurement electrode without placing the cells on the reference electrode.

## Description

### BACKGROUND OF THE INVENTION

### 1. Field of the Invention

The technology of the present disclosure relates to a cell seeding apparatus, an information processing device, and an information processing method.

### 2. Description of the Related Art

In order to improve an efficiency of new drug development, for example, a method of performing a toxicity evaluation using cells such as myocardial cells and nerve cells produced from induced pluripotent stem (iPS) cells has been developed (see, for example, WO2019/131806A). The toxicity evaluation is performed by evaluating responsiveness of cells to a drug.

As a culture vessel for the cells, for example, a well plate in which a plurality of wells are formed is used. A microelectrode array (MEA) in which a plurality of minute measurement electrodes are disposed is provided on a bottom surface of each well. In addition, a reference electrode to which a reference potential is applied is provided around the microelectrode array on the bottom surface of each well (for example, see JP2021-179319A).

Such a well plate is called an MEA plate. A waveform (for example, a myocardial waveform indicating pulsation of the myocardial cells) indicating an electrophysiological change of cells cultured in the wells is output from each measurement electrode of the microelectrode array. The toxicity evaluation is performed by measuring a change in waveform with respect to a drug.

The cells are seeded on the MEA plate by dropping a cell suspension to each well using a pipette or the like.

### SUMMARY OF THE INVENTION

In order to accurately measure a waveform with the microelectrode array, it is necessary to accurately seed the cells on the measurement electrode. However, the measurement electrode has a small diameter of several tens of micrometers, whereas liquid droplets of the cell suspension produced in a case in which the cells are manually seeded using a pipette have a large diameter of several millimeters. Therefore, in a case in which seeding is manually performed, there is a problem in that a positional accuracy of the seeding is low, and the cells cannot be accurately seeded on the measurement electrode.

In addition, since the reference electrode is an electrode to which the reference potential is applied, it is preferable that no cells are placed on the reference electrode. However, in a case in which seeding is manually performed, a positional accuracy of the seeding is low, so that it is not easy to place the cells on the measurement electrode without placing the cells on the reference electrode.

An object of the technology of the present disclosure is to provide a cell seeding apparatus, an information processing device, and an information processing method that enable cells to be seeded accurately.

In order to achieve the above object, a cell seeding apparatus of the present disclosure comprises: a dropping device that drops a liquid droplet of a cell suspension into a cell culture vessel having a measurement electrode and a reference electrode disposed on a bottom surface; an imaging device that is provided in the dropping device and that images the bottom surface to generate an image; and an information processing device that detects positions of the measurement electrode and the reference electrode based on the image and that decides, based on detection information, a dropping position of the liquid droplet at which cells are to be placed on the measurement electrode without placing the cells on the reference electrode.

It is preferable that the information processing device decides the dropping position and a dropping amount of the liquid droplet based on the detection information.

It is preferable that the information processing device decides the dropping amount such that the dropped liquid droplet does not expand and reach the reference electrode, based on a relationship between the dropping amount and a post-expansion size of the liquid droplet.

It is preferable that the information processing device presents a post-expansion size of the liquid droplet assumed based on the decided dropping amount to a user and changes the dropping amount based on an operation by the user.

It is preferable that the imaging device is an image sensor that performs imaging with visible light or a laser type sensing device.

It is preferable that the reference electrode is disposed around a microelectrode array in which a plurality of the measurement electrodes are arranged in a two-dimensional array form.

It is preferable that the cells are myocardial cells or nerve cells.

An information processing device of the present disclosure is an information processing device that decides a dropping amount of a liquid droplet of a cell suspension to be dropped by a dropping device into a cell culture vessel having a measurement electrode and a reference electrode disposed on a bottom surface, the information processing device comprising: a processor, in which the processor detects positions of the measurement electrode and the reference electrode based on an image generated by imaging the bottom surface with an imaging device and decides, based on detection information, a dropping position of the liquid droplet at which cells are to be placed on the measurement electrode without placing the cells on the reference electrode.

An information processing method of the present disclosure is an information processing method of deciding a dropping amount of a liquid droplet of a cell suspension to be dropped by a dropping device into a cell culture vessel having a measurement electrode and a reference electrode disposed on a bottom surface, the information processing method comprising: detecting positions of the measurement electrode and the reference electrode based on an image generated by imaging the bottom surface with an imaging device and deciding, based on detection information, a dropping position of the liquid droplet at which cells are to be placed on the measurement electrode without placing the cells on the reference electrode.

According to the technology of the present disclosure, it is possible to provide a cell seeding apparatus, an information processing device, and an information processing method that enable cells to be seeded accurately.

### BRIEF DESCRIPTION OF THE DRAWINGS

Fig. 1 is a diagram schematically showing a configuration of a cell seeding apparatus.
Fig. 2 is a perspective view showing an example of an MEA plate.
Fig. 3 is a diagram showing a configuration example of a bottom surface of a well.
Fig. 4 is a block diagram showing an example of a configuration of an information processing device.
Fig. 5 is a block diagram showing an example of functions configured in a processor and a controller.
Fig. 6 is a diagram showing an example of a dropping position decided by a dropping position decision unit.
Fig. 7 is a flowchart showing an example of a flow of processing of the cell seeding apparatus.
Fig. 8 is a diagram showing an example of the bottom surface of the well into which liquid droplets have been dropped.
Fig. 9 is a diagram showing an evaluation flow of a toxicity evaluation.
Fig. 10 is a block diagram showing functions configured in a processor and a controller according to a first modification example.
Fig. 11 is a diagram describing a shortest distance between a measurement electrode and a reference electrode.
Fig. 12 is a diagram showing an example of a reference table.
Fig. 13 is a block diagram showing functions configured in a processor and a controller according to a second modification example.
Fig. 14 is a diagram showing an example of a GUI screen.
Fig. 15 is a flowchart showing an example of a flow of dropping amount change processing.
Fig. 16 is a diagram showing a plurality of assumed regions in consideration of variations.
Fig. 17 is a diagram showing a modification example of the GUI screen.

### DESCRIPTION OF THE PREFERRED EMBODIMENTS

Hereinafter, an embodiment according to the technology of the present disclosure will be described with reference to the drawings. In the present disclosure, a vertical direction is defined as a Z direction, and one direction orthogonal to the Z direction is defined as an X direction. In addition, a direction orthogonal to both the Z direction and the X direction is defined as a Y direction.

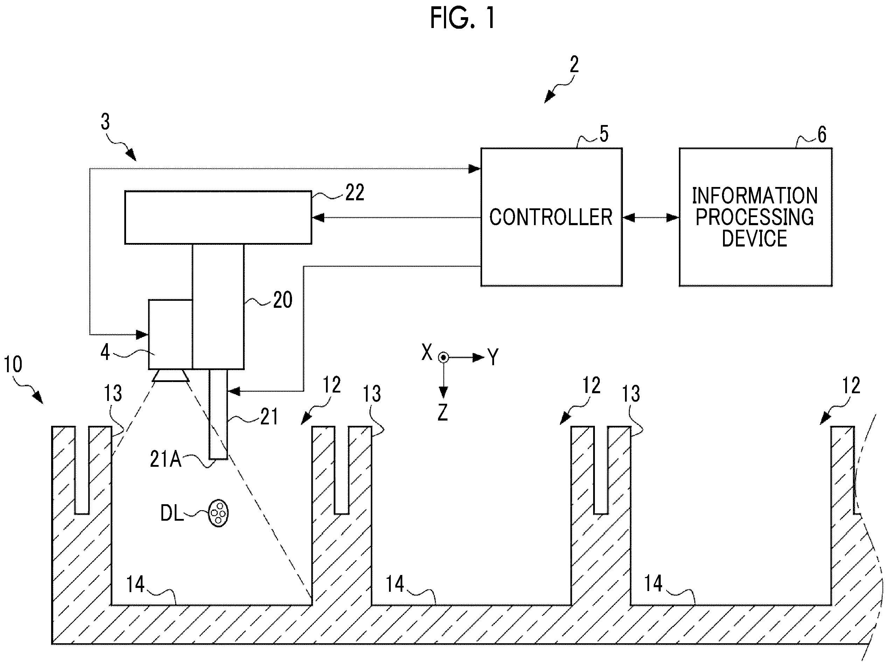

Fig. 1 schematically shows a configuration of a cell seeding apparatus 2. The cell seeding apparatus 2 is an apparatus that seeds cells by dropping a cell suspension to each of a plurality of culture wells (hereinafter, simply referred to as wells) 12 formed on an MEA plate 10. In the present embodiment, nerve cells or myocardial cells produced from iPS cells are used as the cells.

The MEA plate 10 is mounted on a potential measurement device (not shown) after the cells are seeded by the cell seeding apparatus 2 and the cells are cultured by a culture device (not shown). In the potential measurement device, a waveform (for example, a myocardial waveform indicating pulsation of the myocardial cells) indicating an electrophysiological change in the cultured cell group is measured.

The cell seeding apparatus 2 is configured of a dispenser 3, an imaging device 4, a controller 5, and an information processing device 6. The dispenser 3 is configured of an accommodation part 20, a dropping part 21, and a moving mechanism 22. The dispenser 3 is an example of a "dropping device" according to the technology of the present disclosure.

The accommodation part 20 is a tank that accommodates a cell suspension in which cells are suspended in a liquid medium. The dropping part 21 is connected to a lower end of the accommodation part 20.

The dropping part 21 drops the cell suspension accommodated in the accommodation part 20 toward the well 12 as a liquid droplet DL. The dropping part 21 is, for example, a nozzle having a circular cross-sectional shape and stretched in the Z direction. The dropping part 21 jets the liquid droplet DL in the Z direction from a nozzle opening 21A by means of, for example, a piezoelectric method similar to that of an inkjet that jets ink particles using a piezoelectric element. The liquid droplet DL contains one or more cells.

The moving mechanism 22 is connected to an upper end of the accommodation part 20. The moving mechanism 22 moves the accommodation part 20 and the dropping part 21 in the X direction, the Y direction, and the Z direction. That is, the moving mechanism 22 displaces the dropping part 21 relative to the well 12.

The imaging device 4 is, for example, connected to the accommodation part 20 and moves integrally with the accommodation part 20 and the dropping part 21. The imaging device 4 images a bottom surface 14 of the well 12 into which the liquid droplet DL is dropped (that is, the cells are seeded) to generate an image. For example, the imaging device 4 is an image sensor that performs imaging with visible light. A light source that irradiates the bottom surface 14 of the well 12 with illumination light may be provided. The imaging device 4 may be of an epi-illumination type or a transmission type.

The controller 5 performs a dropping control of dropping the liquid droplet DL by driving the piezoelectric element of the dropping part 21, a movement control of moving the dropping part 21 by driving the moving mechanism 22, and an imaging control of causing the imaging device 4 to perform the imaging operation.

The information processing device 6 is configured of a general computer such as a personal computer. The information processing device 6 detects an electrode position in the well 12 and decides a dropping position based on the image acquired by the imaging device 4. The information processing device 6 gives an instruction to the controller 5 to drop the liquid droplet DL at the decided dropping position.

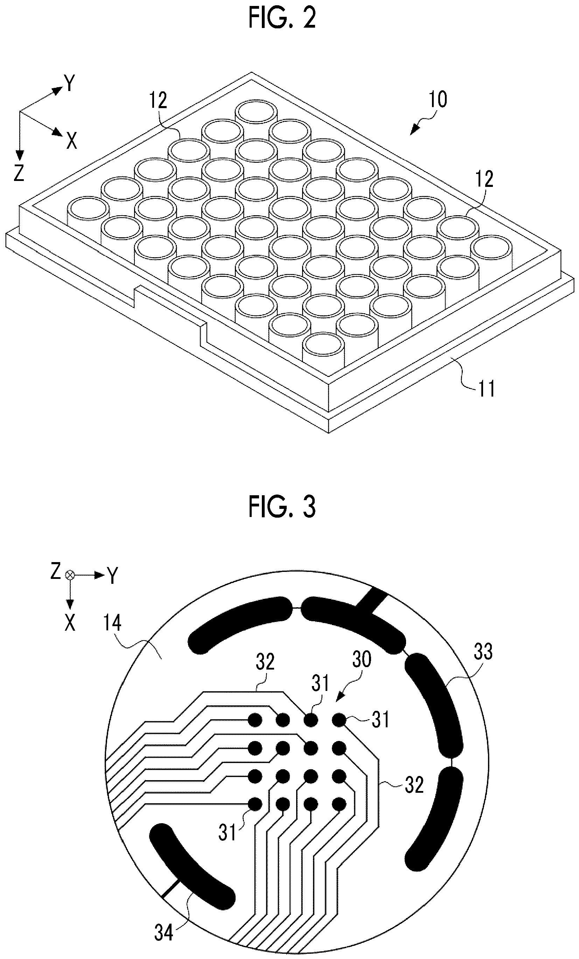

Fig. 2 shows an example of the MEA plate 10. The MEA plate 10 is a multi-well plate in which the plurality of the wells 12 are arranged on a substrate 11. The MEAplate 10 shown in Fig. 2 has 48 wells 12. The number of the wells 12 provided in the MEA plate 10 is not limited to 48, and may be 24, 96, or the like.

The well 12 is a substantially cylindrical vessel with an opening 13 (see Fig. 1) formed at an upper part. The well 12 is an example of a "cell culture vessel" according to the technology of the present disclosure.

Fig. 3 shows a configuration example of the bottom surface 14 of the well 12. A microelectrode array 30 is embedded in the bottom surface 14 of the well 12. The microelectrode array 30 has a plurality of measurement electrodes 31 arranged in a two-dimensional array form. In the example shown in Fig. 3, the microelectrode array 30 has 16 measurement electrodes 31 arranged in a 4 × 4 square pattern. The measurement electrode 31 is exposed on the bottom surface 14 of the well 12 and comes into contact with the seeded cells. The measurement electrode 31 has a diameter of several tens of micrometers. Each of the measurement electrodes 31 is connected to a potential measurement circuit of a potential measurement device via a wiring line 32.

In addition to the microelectrode array 30, a reference electrode 33 is provided on the bottom surface 14 of the well 12. The reference electrode 33 is disposed around the microelectrode array 30 along an outer edge of the bottom surface 14 so as not to come into contact with the cells to be measured. A ground potential as a reference potential is applied to the reference electrode 33. The extracellular potential is measured from a potential difference between the measurement electrode 31 and the reference electrode 33. One cell to be seeded is smaller than a size of the measurement electrode 31. A plurality of cells (approximately 2 to 3 cells) are seeded on one measurement electrode 31.

In addition, a stimulation electrode 34 for applying a stimulus to the cells is provided on the bottom surface 14 of the well 12. Note that it is not essential to apply a stimulus to the cells, and the stimulation electrode 34 does not have to be provided in the well 12.

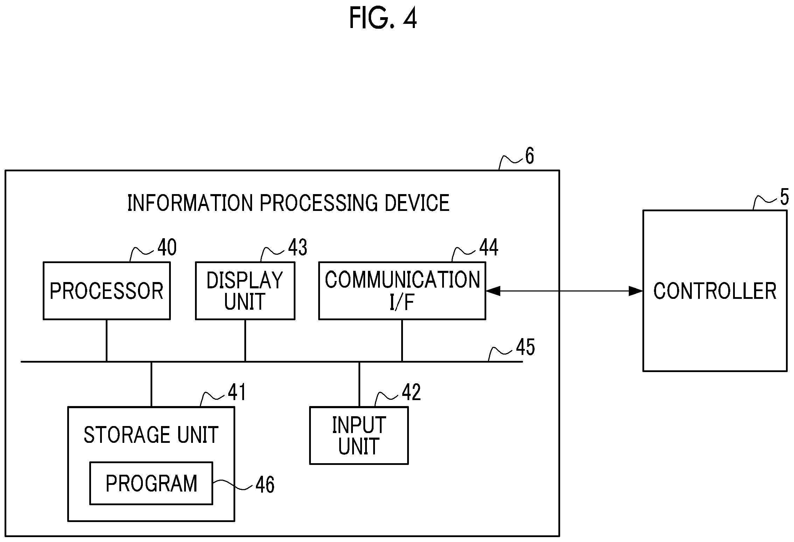

Fig. 4 shows an example of a configuration of the information processing device 6. The information processing device 6 includes a processor 40, a storage unit 41, an input unit 42, a display unit 43, a communication I/F 44, and a bus 45. The processor 40 is a computer that realizes various functions by reading out a program 46 and various kinds of data stored in the storage unit 41 and executing processing. The processor 40 is, for example, a central processing unit (CPU).

The storage unit 41 is a storage device that stores the program 46 and the various kinds of data in a case in which the processor 40 executes processing. The storage unit 41 includes, for example, a random access memory (RAM), a read only memory (ROM), or a storage. The RAM is, for example, a volatile memory used as a work area or the like of the processor 40. The ROM is, for example, a non-volatile memory such as a flash memory that holds the program 46 and various kinds of data. The storage is, for example, a large-capacity storage device such as a hard disk drive (HDD) or a solid state drive (SSD), and stores an operating system (OS), various kinds of data, and the like. The storage unit 41 may be configured as an external apparatus connected to the information processing device 6.

The input unit 42 is an input device such as a keyboard, a touch pad, or a mouse. The display unit 43 is a display device such as a liquid crystal display. The input unit 42 and the display unit 43 may be configured as external devices connected to the information processing device 6. The processor 40 communicates with the controller 5 via the communication I/F 44.

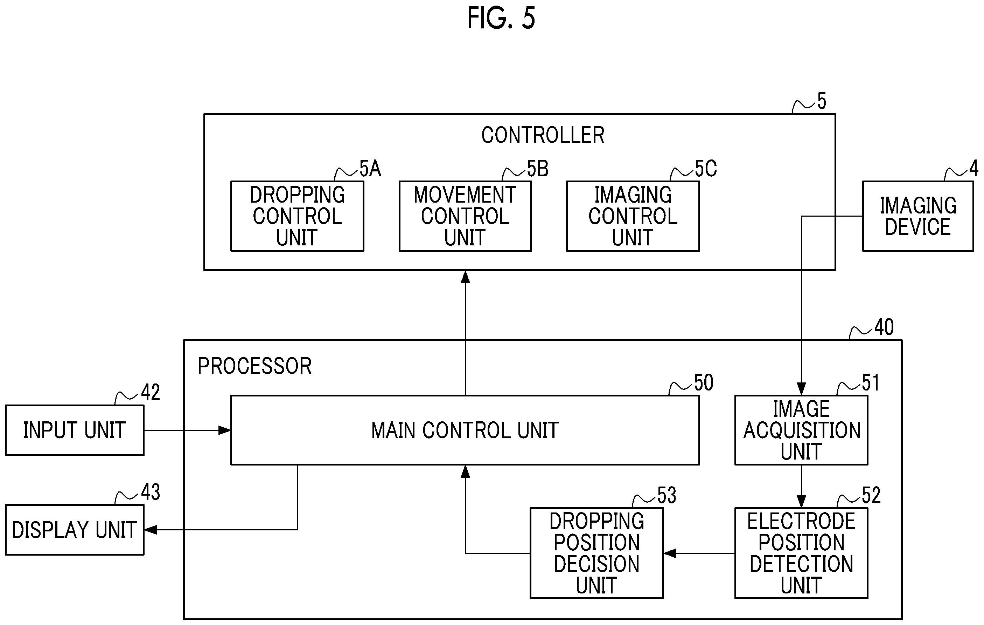

Fig. 5 shows an example of functions configured in the processor 40 and the controller 5. The controller 5 is configured of a dropping control unit 5A that performs the above-described dropping control, a movement control unit 5B that performs the above-described movement control, and an imaging control unit 5C that performs the above-described imaging control. These may be configured using either hardware or software. In addition, the controller 5 may be incorporated into the information processing device 6.

The processor 40 functions as a main control unit 50, an image acquisition unit 51, an electrode position detection unit 52, and a dropping position decision unit 53. These functions are realized by the processor 40 executing processing based on the program 46 (see Fig. 4). These functions may be realized by hardware.

The main control unit 50 comprehensively controls various functions in the controller 5 and the processor 40. In addition, the main control unit 50 receives an operation signal input from the input unit 42 and performs a display control of displaying various types of information on the display unit 43.

The main control unit 50 gives an instruction to the imaging control unit 5C to cause the imaging device 4 to image the bottom surface 14 of the well 12 before the dispenser 3 drops the liquid droplet DL into the well 12. The image acquisition unit 51 acquires the image generated by the imaging device 4.

The electrode position detection unit 52 detects positions of the plurality of measurement electrodes 31 and the reference electrode 33 based on the image acquired by the image acquisition unit 51, and inputs detection information to the dropping position decision unit 53. Since the positions of the plurality of measurement electrodes 31 and the reference electrode 33 vary for each well 12, the electrode position detection unit 52 detects their exact positions.

The dropping position decision unit 53 decides the dropping position of the liquid droplet DL based on the detection information input from the electrode position detection unit 52. Specifically, the dropping position decision unit 53 decides a position at which cells are to be placed on the measurement electrode 31 without placing the cells on the reference electrode 33, as the dropping position. Since there is a possibility that placing the cells on the reference electrode 33 to which the reference potential is applied may affect a measurement waveform by the potential measurement device, such as causing an offset, the electrode position detection unit 52 decides the dropping position such that the cells are not placed on the reference electrode 33.

Based on the dropping position decided by the dropping position decision unit 53, the main control unit 50 gives an instruction to the movement control unit 5B to dispose the dropping part 21 above the dropping position, and then gives an instruction to the dropping control unit 5A to drop the liquid droplet DL at the dropping position.

The electrode position detection unit 52 may detect a position of the stimulation electrode 34 in addition to the positions of the plurality of measurement electrodes 31 and the reference electrode 33. In this case, the dropping position decision unit 53 decides a position at which cells are to be placed on the measurement electrode 31 without placing the cells on the reference electrode 33 and the stimulation electrode 34, as the dropping position. This is because, in a case in which cells are placed on the stimulation electrode 34, the stimulus applied from the stimulation electrode 34 to the cells may not have a desired intensity, which may affect the measurement waveform by the potential measurement device.

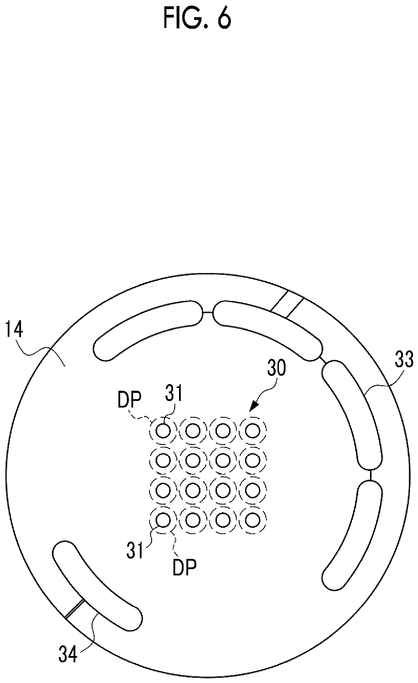

Fig. 6 shows an example of the dropping position decided by the dropping position decision unit 53. As shown in Fig. 6, the dropping position decision unit 53 decides a dropping position DP on each measurement electrode 31 based on the detection information of the electrode position such that the cells are not placed on the reference electrode 33.

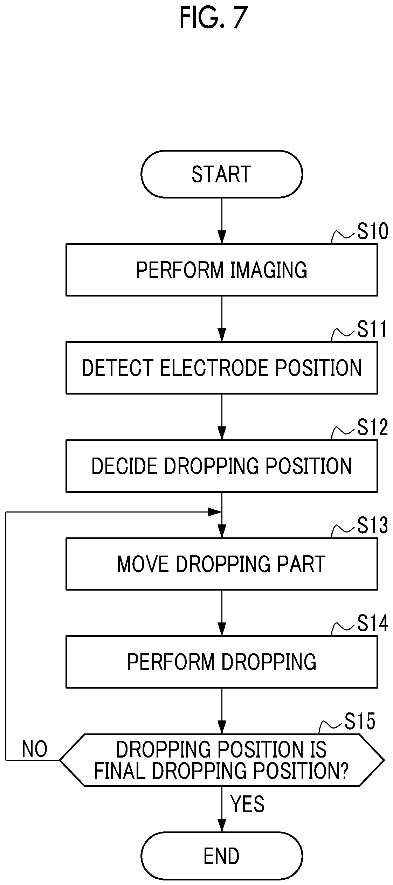

Fig. 7 shows an example of a flow of processing of the cell seeding apparatus 2. Fig. 7 shows a flow of processing in a case in which cells are seeded in one well 12.

In a case in which the main control unit 50 receives a start instruction from the input unit 42 in a state in which the MEA plate 10 is set in the cell seeding apparatus 2, the main control unit 50 gives an instruction to the imaging control unit 5C to cause the imaging device 4 to image the bottom surface 14 of the well 12 (step S10). The image generated by the imaging device 4 is transmitted to the information processing device 6 via the controller 5.

In the information processing device 6, the image transmitted from the imaging device 4 is acquired by the image acquisition unit 51, and the electrode position is detected by the electrode position detection unit 52 based on the image acquired by the image acquisition unit 51 (step S11). Then, the dropping position DP is decided by the dropping position decision unit 53 based on the detection information of the electrode position detected by the electrode position detection unit 52 (step S12). In the present embodiment, a plurality of the dropping positions DP are decided by the dropping position decision unit 53.

The main control unit 50 gives an instruction to the movement control unit 5B to move the dropping part 21 so that the dropping part 21 is positioned above one dropping position DP among the plurality of dropping positions DP (step S 13). Then, the main control unit 50 gives an instruction to the dropping control unit 5A to drop the liquid droplet DL (step S14).

The main control unit 50 determines whether the dropping position DP at which the liquid droplet DL is dropped in step S14 is a final dropping position (that is, whether the dropping has been performed at all the plurality of dropping positions DP decided by the dropping position decision unit 53) (step S15).

In a case in which it is determined that the dropping position DP is not the final dropping position (step S15: NO), the main control unit 50 returns the processing to step S13 and moves the dropping part 21 above the next dropping position DP. Steps S13 and S 14 are repeatedly executed until it is determined in step S15 that the dropping position DP is the final dropping position. The main control unit 50 ends the processing in a case in which it is determined that the dropping position DP is the final dropping position (step S15: YES).

The main control unit 50 performs the processing of steps S10 to S15 with respect to all the wells 12 included in the MEA plate 10 while changing the well 12 as a seeding target.

As described above, according to the technology of the present disclosure, the electrode position is detected based on the image obtained by imaging the bottom surface 14 of the well 12, and the dropping position is decided based on the detection information of the electrode position, so that the cells can be seeded accurately.

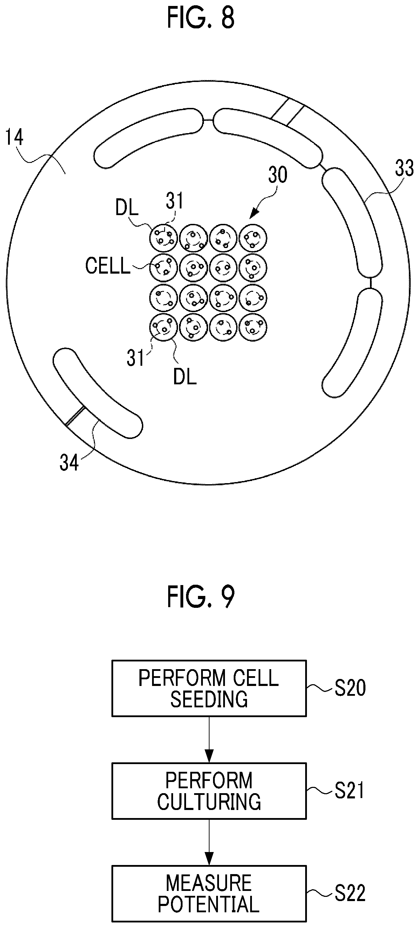

Fig. 8 shows an example of the bottom surface 14 of the well 12 into which the liquid droplet DL is dropped by the cell seeding apparatus 2. Each liquid droplet DL contains one or more cells. The liquid droplet DL, after being dropped onto the bottom surface 14, flattens by its own weight, and its planar shape becomes large (that is, expands). In addition, the cells contained in the liquid droplet DL are adherent cells and adhere to the measurement electrode 31.

Fig. 9 shows an evaluation flow of the toxicity evaluation. In the toxicity evaluation, first, the MEA plate 10 is set in the cell seeding apparatus 2, and cells are seeded in each well 12 (step S20). Next, the MEA plate 10 is taken out from the cell seeding apparatus 2, and after the culture solution is injected into each well 12, the cells are cultured by the culture device (step S21). The cells are cultured for a period of about 1 week. Through the culturing, the seeded cells come out of hibernation, and a plurality of cells are bonded to each other to form a cell group (for example, a spontaneously pulsating myocardial sheet) exhibiting a single behavior.

Next, the MEA plate 10 is taken out from the culture device, and the extracellular potential is measured by the potential measurement device (step S22). In step S22, a drug candidate compound is added to the culture solution, and the toxicity evaluation is performed by analyzing a change in the waveform before and after the addition of the drug candidate compound.

Next, various modification examples of the above embodiment will be described.

### [First Modification Example]

In the above-described embodiment, the dropping position is decided based on the detection information of the electrode position, but in a first modification example, a dropping amount is decided in addition to the dropping position.

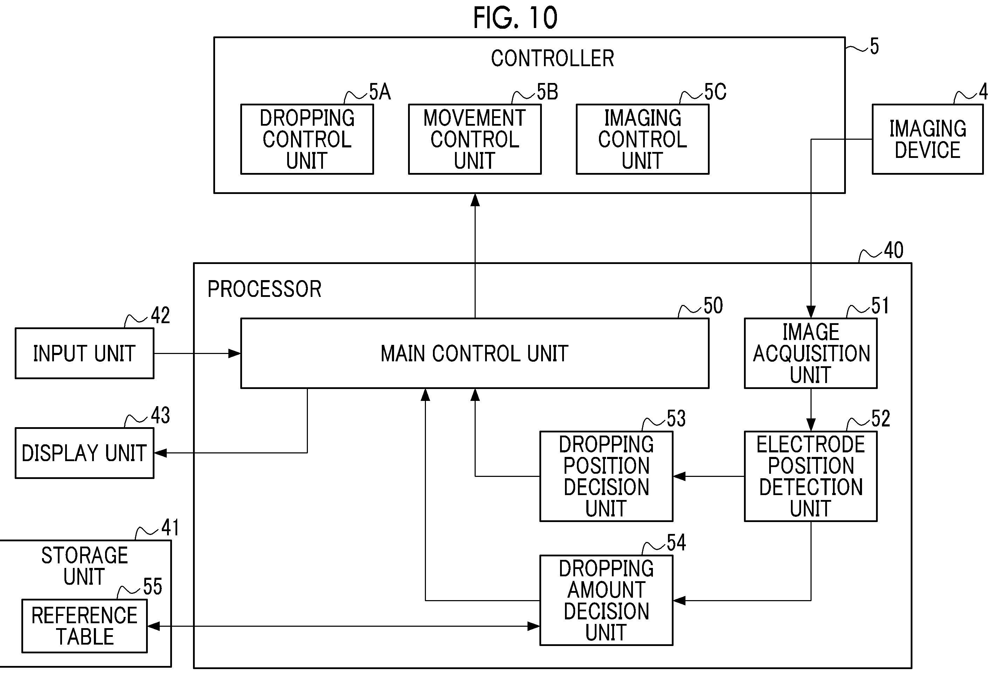

Fig. 10 shows functions configured in the processor 40 and the controller 5 according to the first modification example. In the present modification example, the processor 40 realizes a dropping amount decision unit 54 in addition to the main control unit 50, the image acquisition unit 51, the electrode position detection unit 52, and the dropping position decision unit 53. The dropping amount decision unit 54 decides the dropping amount of the liquid droplet DL (that is, the volume of the liquid droplet DL) by the dropping part 21 based on the detection information of the electrode position detected by the electrode position detection unit 52.

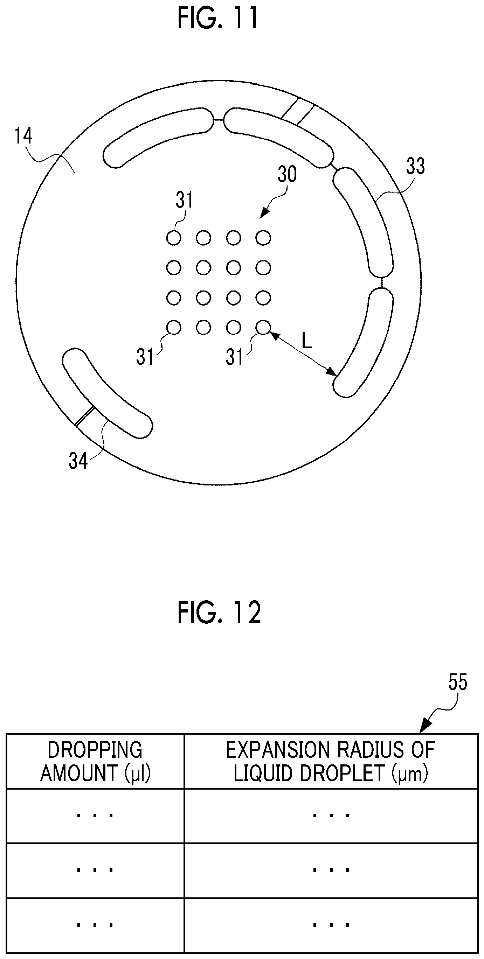

Specifically, as shown in Fig. 11, the dropping amount decision unit 54 calculates the shortest distance L between the measurement electrode 31 and the reference electrode 33 based on the detection information of the electrode position. Then, the dropping amount decision unit 54 decides the liquid droplet amount such that the dropped liquid droplet DL does not expand and reach the reference electrode 33, based on the shortest distance L.

In the present modification example, the storage unit 41 stores a reference table 55. The reference table 55 stores a relationship between the dropping amount and the post-expansion size of the liquid droplet DL. In the present embodiment, as shown in Fig. 12, the dropping amount and the post-expansion radius (hereinafter, referred to as the expansion radius) of the liquid droplet DL are stored. After calculating the shortest distance L, the dropping amount decision unit 54 refers to the reference table 55 to decide the dropping amount such that the expansion radius of the liquid droplet DL occupies a certain proportion of the shortest distance L.

In the present modification example, the main control unit 50 controls the piezoelectric element of the dropping part 21 via the dropping control unit 5A, thereby setting the volume of the liquid droplet DL to be dropped from the dropping part 21 to the dropping amount decided by the dropping amount decision unit 54.

Other configurations of the cell seeding apparatus according to the first modification example are the same as the configurations of the cell seeding apparatus 2 according to the above-described embodiment.

According to the present modification example, the dropping amount is decided in addition to the dropping position based on the detection information of the electrode position, so that the cells can be seeded accurately.

A certain degree of variation occurs in the expansion radius of the liquid droplet DL after being actually dropped by the dispenser 3. This is due to a variation in the amount of the liquid droplet DL dropped by the dispenser 3. Therefore, the reference table 55 may store information (for example, standard deviation) representing the variation in the expansion radius in addition to the expansion radius (average value) of the liquid droplet DL. In this case, the dropping amount decision unit 54 decides the dropping amount in consideration of the variation in the expansion radius. For example, the dropping amount decision unit 54 decides the dropping amount such that a value obtained by adding the standard deviation to the expansion radius (average value) occupies a certain proportion of the shortest distance L.

In addition, the expansion radius of the liquid droplet DL varies depending on a contact angle between the liquid droplet DL and the bottom surface 14 of the well 12. The contact angle refers to an angle between a tangent line of the liquid droplet DL and the bottom surface 14. The smaller the contact angle, the larger the expansion radius. The contact angle varies depending on the main component of the cell suspension, the type of coating on the bottom surface 14, or the like. Therefore, the reference table 55 may store a plurality of expansion radii corresponding to information related to the contact angle. In addition, the information related to the contact angle may be input via the input unit 42, and the main control unit 50 may correct the expansion radius stored in the reference table 55 based on the input information.

The expansion radius is not limited to the data stored in the reference table 55 and may be defined as a function that depends on the dropping amount. In addition, the expansion radius may be defined as a function that depends on information related to the dropping amount and the contact angle.

### [Second Modification Example]

In the first modification example, the dropping amount is decided in addition to the dropping position based on the detection information of the electrode position, but in a second modification example, the decided dropping amount is further changeable by the user.

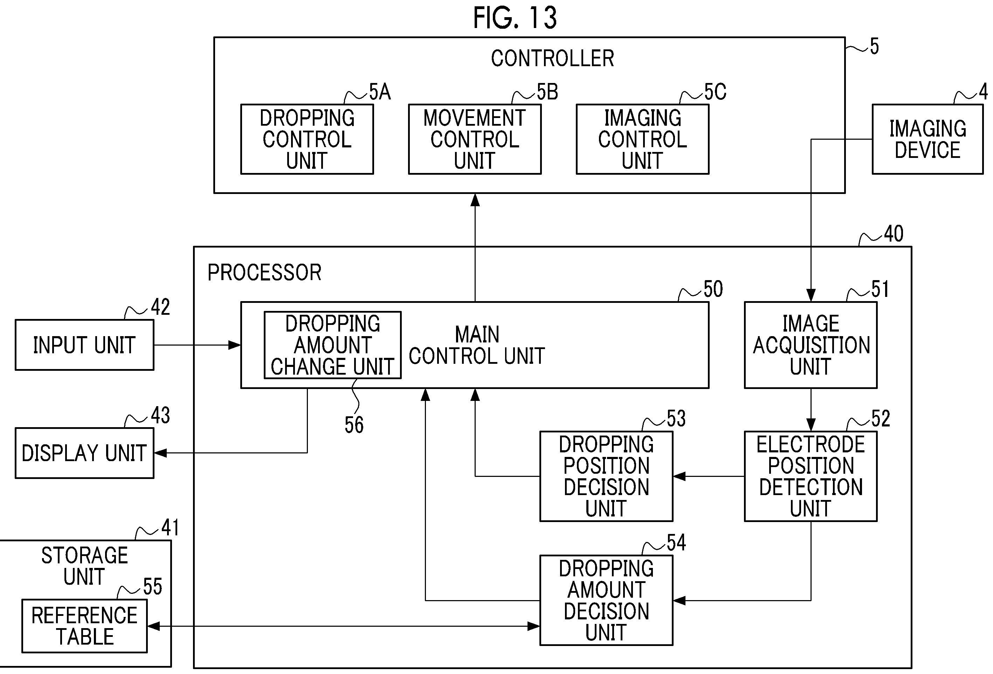

Fig. 13 shows functions configured in the processor 40 and the controller 5 according to the second modification example. The second modification example is different from the first modification example in that a dropping amount change unit 56 is provided in the main control unit 50.

The dropping amount change unit 56 acquires the dropping amount decided by the dropping amount decision unit 54 and presents the post-expansion size of the liquid droplet DL assumed based on the decided dropping amount to the user via the display unit 43. The user can change the dropping amount using the input unit 42. In a case in which an operation of changing the dropping amount is performed, the dropping amount change unit 56 changes the dropping amount to be dropped by the dropping part 21.

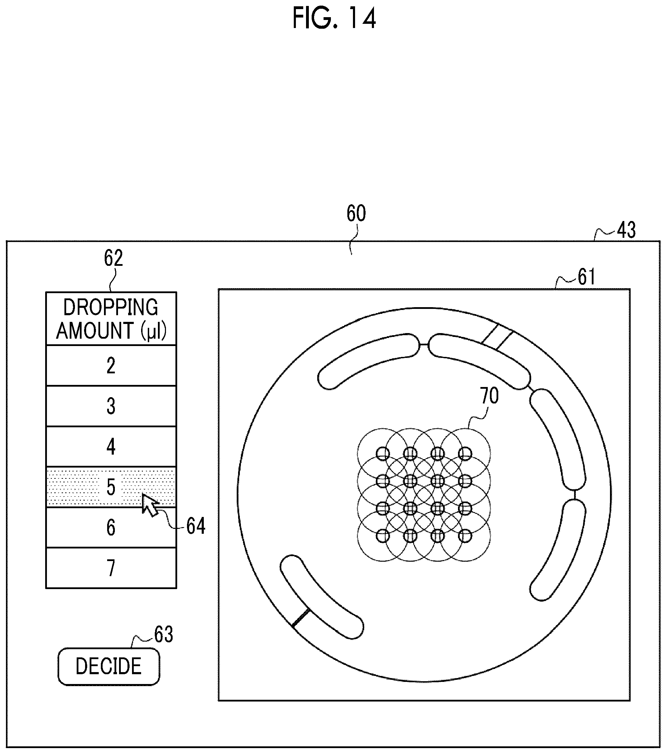

Fig. 14 shows an example of a graphical user interface (GUI) screen 60 displayed on the display unit 43. In order to present the assumed post-expansion size of the liquid droplet DL and to change the dropping amount, the dropping amount change unit 56 displays the GUI screen 60 shown in Fig. 14 on the display unit 43. The GUI screen 60 is configured of an image display region 61, a change operation region 62, and a decision button 63.

The dropping amount change unit 56 displays the image acquired by the image acquisition unit 51 in the image display region 61. In addition, the dropping amount change unit 56 displays a post-expansion region (hereinafter, referred to as an assumed region) 70 of the liquid droplet DL assumed based on the dropping amount decided by the dropping amount decision unit 54. In the present modification example, the assumed region 70 is circular. The dropping amount change unit 56 decides the size of the assumed region 70 based on the expansion radius of the liquid droplet DL acquired by the dropping amount decision unit 54 from the reference table 55.

The dropping amount change unit 56 displays a plurality of dropping amounts including the dropping amount decided by the dropping amount decision unit 54, in the change operation region 62. The user can perform a selection operation of selecting the dropping amount by operating a cursor 64. In a case in which the dropping amount is selected, the dropping amount change unit 56 changes the size of the assumed region 70 to a size corresponding to the selected dropping amount. The user can select the dropping amount to make the assumed region 70 a desired size while checking the size of the assumed region 70. The user can perform a decision operation of deciding the final dropping amount by clicking the decision button 63.

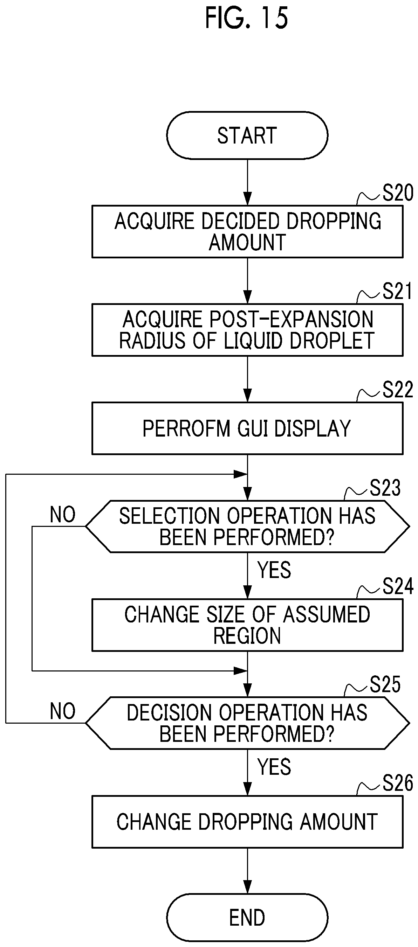

Fig. 15 shows an example of a flow of the dropping amount change processing by the dropping amount change unit 56. First, the dropping amount change unit 56 acquires the dropping amount decided by the dropping amount decision unit 54 (step S20). In addition, the dropping amount change unit 56 acquires the expansion radius of the liquid droplet DL acquired by the dropping amount decision unit 54 from the reference table 55 in a case of deciding the dropping amount (step S21).

The dropping amount change unit 56 generates the GUI screen 60 shown in Fig. 14 based on the liquid droplet amount acquired in step S20 and the expansion radius of the liquid droplet DL acquired in step S21, and displays the GUI screen 60 on the display unit 43 (step S22). The dropping amount change unit 56 determines whether the user has performed the selection operation of selecting the dropping amount based on the GUI screen 60 (step S23).

In a case in which the selection operation has been performed (step S23: YES), the dropping amount change unit 56 changes the size of the assumed region 70 to a size corresponding to the selected dropping amount (step S24). In a case in which the selection operation has not been performed (step S23: NO), the dropping amount change unit 56 determines whether or not the decision operation has been performed (step S25).

In a case in which the decision operation has not been performed (step S25: NO), the dropping amount change unit 56 returns the processing to step S23. In a case in which the decision operation has been performed (step S25: YES), the dropping amount change unit 56 changes the dropping amount (step S26), and ends the processing. The dropping amount change unit 56 does not change the liquid droplet amount in a case in which the liquid droplet amount selected by the user is the dropping amount decided by the dropping amount decision unit 54.

In the present modification example, the main control unit 50 controls the piezoelectric element of the dropping part 21 via the dropping control unit 5A, thereby setting the volume of the liquid droplet DL to be dropped from the dropping part 21 to the dropping amount changed by the dropping amount change unit 56.

According to the present modification example, the dropping amount is decided in addition to the dropping position based on the detection information of the electrode position, and the user can change the dropping amount, so that the cells can be seeded accurately.

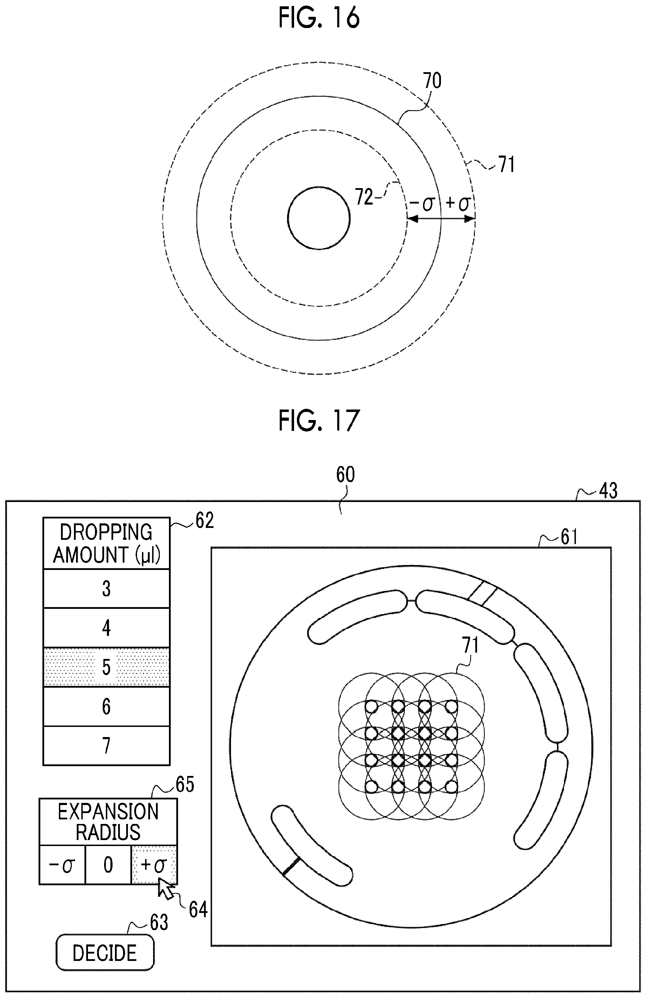

In addition, as described above, in a case in which the reference table 55 stores information (for example, standard deviation) representing the variation in the expansion radius in addition to the expansion radius (average value) of the liquid droplet DL, an assumed region of a size in consideration of the variation may be displayed on the display unit 43. For example, as shown in Fig. 16, the assumed region 70 corresponding to the expansion radius (average value), an assumed region 71 corresponding to a radius obtained by adding a standard deviation δ to the expansion radius (average value), and an assumed region 72 corresponding to a radius obtained by subtracting the standard deviation δ from the expansion radius (average value) may be displayed on the display unit 43.

In addition, the user may select which of the assumed regions 70, 71, and 72 to display. For example, as shown in Fig. 17, a selection operation region 65 for selecting the variation in the expansion radius is provided on the GUI screen 60. The main control unit 50 selects the assumed region 70 in a case in which "0" is clicked, selects the assumed region 71 in a case in which "+σ" is clicked, and selects the assumed region 72 in a case in which "-σ" is clicked. Fig. 17 illustrates a case in which "+σ" is clicked. As a result, the user can decide the dropping amount while checking a degree of the variation in the expansion radius.

### [Other Modification Examples]

In the above-described embodiment and each modification example, the imaging device 4 is used as an image sensor that performs imaging with visible light. Alternatively, the imaging device 4 may be a laser type sensing device that performs imaging by irradiating the bottom surface 14 of the well 12 with laser light and receiving reflected light of the laser light reflected by the electrodes including the measurement electrode 31 and the reference electrode 33.

In the above-described embodiment and each modification example, the dispenser 3 is of a piezoelectric type, but the present disclosure is not limited to the piezoelectric type, and a dispenser of a pressurization type or the like may be used. In addition, the dropping device of the present disclosure includes a so-called bio 3D printer.

A hardware structure of a processing unit that executes various kinds of processing, such as the main control unit 50, the image acquisition unit 51, the electrode position detection unit 52, the dropping position decision unit 53, the dropping amount decision unit 54, and the dropping amount change unit 56, is, for example, various processors as shown below.

Various processors include a CPU, a programmable logic device (PLD), a dedicated electric circuit, and the like. As is well known, the CPU is a general-purpose processor that executes software (program) and functions as various processing units. The PLD is a processor whose circuit configuration can be changed after manufacturing, such as a field programmable gate array (FPGA). The dedicated electric circuit is a processor that has a dedicated circuit configuration designed to perform a specific process, such as an application specific integrated circuit (ASIC).

One processing unit may be configured of one of these various processors, or may be configured of a combination of two or more processors of the same type or different types (for example, a combination of a plurality of FPGAs or a combination of a CPU and an FPGA). In addition, a plurality of processing units may be constituted by one processor. As an example in which the plurality of processing units are constituted by one processor, first, one processor is constituted by a combination of one or more CPUs and software and this processor functions as the plurality of processing units. Second, as typified by a system on chip (SoC) or the like, a processor that realizes the functions of the entire system including the plurality of processing units by using one IC chip is used. As described above, the various processing units are configured using one or more of the various processors as a hardware structure.

More specifically, the hardware structure of these various processors is an electric circuit (circuitry) in which circuit elements such as semiconductor elements are combined.

The present disclosure is not limited to the embodiment described above and may adopt various configurations without departing from the spirit of the present disclosure. In addition to the program, the present disclosure extends to a computer-readable storage medium that stores the program in a non-temporary manner.

It is possible to grasp the following technologies by the above description.

### [Appendix 1]

A cell seeding apparatus comprising:
a dropping device that drops a liquid droplet of a cell suspension into a cell culture vessel having a measurement electrode and a reference electrode disposed on a bottom surface;
an imaging device that is provided in the dropping device and that images the bottom surface to generate an image; and
an information processing device that detects positions of the measurement electrode and the reference electrode based on the image and that decides, based on detection information, a dropping position of the liquid droplet at which cells are to be placed on the measurement electrode without placing the cells on the reference electrode.

### [Appendix 2]

The cell seeding apparatus according to Appendix 1,
in which the information processing device decides the dropping position and a dropping amount of the liquid droplet based on the detection information.

### [Appendix 3]

The cell seeding apparatus according to Appendix 2,
in which the information processing device decides the dropping amount such that the dropped liquid droplet does not expand and reach the reference electrode, based on a relationship between the dropping amount and a post-expansion size of the liquid droplet.

### [Appendix 4]

The cell seeding apparatus according to Appendix 2,
in which the information processing device presents a post-expansion size of the liquid droplet assumed based on the decided dropping amount to a user and changes the dropping amount based on an operation by the user.

### [Appendix 5]

The cell seeding apparatus according to any one of Appendices 1 to 4,
in which the imaging device is an image sensor that performs imaging with visible light or a laser type sensing device.

### [Appendix 6]

The cell seeding apparatus according to any one of Appendices 1 to 5,
in which the reference electrode is disposed around a microelectrode array in which a plurality of the measurement electrodes are arranged in a two-dimensional array form.

### [Appendix 7]

The cell seeding apparatus according to any one of Appendices 1 to 6,
in which the cells are myocardial cells or nerve cells.

### [Appendix 8]

An information processing device that decides a dropping amount of a liquid droplet of a cell suspension to be dropped by a dropping device into a cell culture vessel having a measurement electrode and a reference electrode disposed on a bottom surface, the information processing device comprising:
a processor,
in which the processor detects positions of the measurement electrode and the reference electrode based on an image generated by imaging the bottom surface with an imaging device and decides, based on detection information, a dropping position of the liquid droplet at which cells are to be placed on the measurement electrode without placing the cells on the reference electrode.

### [Appendix 9]

An information processing method of deciding a dropping amount of a liquid droplet of a cell suspension to be dropped by a dropping device into a cell culture vessel having a measurement electrode and a reference electrode disposed on a bottom surface, the information processing method comprising:
detecting positions of the measurement electrode and the reference electrode based on an image generated by imaging the bottom surface with an imaging device and deciding, based on detection information, a dropping position of the liquid droplet at which cells are to be placed on the measurement electrode without placing the cells on the reference electrode.

## Claims

1. A cell seeding apparatus comprising:
a dropping device that drops a liquid droplet of a cell suspension into a cell culture vessel having a measurement electrode and a reference electrode disposed on a bottom surface;
an imaging device that is provided in the dropping device and that images the bottom surface to generate an image; and
an information processing device that detects positions of the measurement electrode and the reference electrode based on the image and that decides, based on detection information, a dropping position of the liquid droplet at which cells are to be placed on the measurement electrode without placing the cells on the reference electrode.

2. The cell seeding apparatus according to claim 1,
wherein the information processing device decides the dropping position and a dropping amount of the liquid droplet based on the detection information.

3. The cell seeding apparatus according to claim 2,
wherein the information processing device decides the dropping amount such that the dropped liquid droplet does not expand and reach the reference electrode, based on a relationship between the dropping amount and a post-expansion size of the liquid droplet.

4. The cell seeding apparatus according to claim 2,
wherein the information processing device presents a post-expansion size of the liquid droplet assumed based on the decided dropping amount to a user and changes the dropping amount based on an operation by the user.

5. The cell seeding apparatus according to claim 1,
wherein the imaging device is an image sensor that performs imaging with visible light or a laser type sensing device.

6. The cell seeding apparatus according to claim 1,
wherein the reference electrode is disposed around a microelectrode array in which a plurality of the measurement electrodes are arranged in a two-dimensional array form.

7. The cell seeding apparatus according to claim 1,
wherein the cells are myocardial cells or nerve cells.

8. An information processing device that decides a dropping amount of a liquid droplet of a cell suspension to be dropped by a dropping device into a cell culture vessel having a measurement electrode and a reference electrode disposed on a bottom surface, the information processing device comprising:
a processor,
wherein the processor detects positions of the measurement electrode and the reference electrode based on an image generated by imaging the bottom surface with an imaging device and decides, based on detection information, a dropping position of the liquid droplet at which cells are to be placed on the measurement electrode without placing the cells on the reference electrode.

9. An information processing method of deciding a dropping amount of a liquid droplet of a cell suspension to be dropped by a dropping device into a cell culture vessel having a measurement electrode and a reference electrode disposed on a bottom surface, the information processing method comprising:
detecting positions of the measurement electrode and the reference electrode based on an image generated by imaging the bottom surface with an imaging device and deciding, based on detection information, a dropping position of the liquid droplet at which cells are to be placed on the measurement electrode without placing the cells on the reference electrode.
